# EUROPEAN PATENT SPECIFICATION

(11) **EP 1 464 287 B1**
(45) Date of publication and mention of the grant of the patent: **30.12.2009**
(21) Application number: 04252018.9
(22) Date of filing: 02.04.2004
(51) Int. Cl.: A61B 17/072

(54) **Surgical device for anastomosis**
Chirurgisches Instrument für Anastomose
Instrument chirurgical pour anastomose

(30) Priority: 02.04.2003 US 405684
(43) Date of publication of application: 06.10.2004
(73) Proprietor: ETHICON ENDO-SURGERY, INC., Cincinnati, OH 45242-2839 (US)
(72) Inventor: Hueil, Charles, Loveland, Ohio 45140 (US); Jenkins, Ed, West Chester, Ohio 45069 (US); Knodel, Timothy, Flagstaff, AZ 86001 (US); Knodel, Bryan, Flagstaff, AZ 86001 (US)
(74) Representative: Tunstall, Christopher Stephen

(56) References cited:
- EP-A- 0 648 475
- US-A- 4 397 311
- US-A- 5 535 935
- US-A- 5 826 776

## Description

### Field of the Invention

The present invention relates, in general, to devices for surgically performing anastomosis of hollow organs or vessels.

### Background of the Invention

Creating an anastomosis, or the surgical formation of a passage between two normally distinct lumens, is a critical step of many surgical procedures. This is true in intestinal cancer in which a portion of the small intestine is removed and the remaining portions are rejoined to form a complete path for the flow of ingesta. An anastomosis of the intestines also occurs in gastric bypass surgery, which is performed to cause weight loss in obese patients. In gastric surgery, a gastric pouch is formed by dissecting the stomach. Repeated applications of a linear cutter are used to separate a small portion of the stomach just distal to the esophagus from the rest of the stomach. The jejunum is then transected distally with a linear cutter, a device that both severs and staples tissue. Thereafter, the distal portion of the jejunum, called the Roux limb, is brought to form an anastomosis with the gastric pouch, often referred to as a gastroenterostomy. The gastroenterostomy can be performed with a linear cutter, circular stapler, or hand sewing. Following the gastroenterostomy, the linear cutter may be used to perform a side to side anastomosis to join the Roux limb to the portion of the jejunum extending below the lower portion of the dissected stomach, often referred to as an enteroenterostomy.

Typical linear cutters have two forks forming an implement on the distal end. One fork contains a cartridge assembly to eject staples and a cutting implement to sever tissue, while the other fork has an anvil containing pockets to form staples into the correct shape for holding tissue. The cartridge assembly generally has a tissue surface, which abuts the tissue to be cut and stapled when the forks are placed on tissue and closed. An example of an endocutter can be found in U.S. Patent 5,673,840 issued on October 7, 1997.

When making a side to side anastomosis using a linear cutter, a surgeon aligns two lumens so that the side walls touch. A small opening is made in each lumen to allow entry of a fork of a linear cutter. One fork of the linear cutter is inserted into one lumen, and the other fork of the linear cutter is inserted into the other lumen. The forks of the linear cutter are then closed so that a portion of one wall of each lumen is compressed between them. The two small entry openings are separated only by these two compressed lumen wall portions. Firing the linear cutter severs and staples tissue along a line extending from the proximal end of tissue in the device to a point near the end of the forks, and creates a cutline that extends radially outwards from the original entry openings between the staple lines. A large, irregularly shaped opening to the outside of the lumens is created. The large opening to the outside of the lumens requires closure while maintaining the desired communication between the two lumens. Closing the opening to the outside of the lumens, while leaving recently formed passageway intact, can be a difficult task requiring much surgical skill and operating room time. The surgeon can have difficulty in keeping the lumens uniform while sewing a large opening, as the lumens tend to plicate and become irregular as the needle passes through the opening. A surgeon may prefer to staple the opening by use of a stapling device applied perpendicular to the first application. While the second stapling has worked adequately, difficulty in positioning the second application of the stapling device can make it difficult to ascertain the size of the resultant passageway.

If the passageway between the two lumens were not to extend proximally into the entry openings made for the members of the linear cutter, only closure of two small entry openings would be required. The lumens would then remain more regular, and the size of the passageway would become more predictable. A linear stapling device creating a cut line that does not extend to the proximal end of the tissue within the working forks is described in US Patent Number 6,066,144 issued to Wolf et al on May 23, 2000. However, Wolf et al's cutting blade moves generally perpendicular to the plane of the tissue compressed in the device, much as a cheesewire cutting cheese. This cutting action, useful for delicate vascular tissue, may not be the optimum action to sever tough bowel and stomach tissue. Cutting generally parallel to the tissue plane, and generally parallel to the tissue surface of the cartridge, presents less tissue area to the blade, and so decreases force needed to cut tough tissue. An exemplary stapling / cutting device is disclosed in US Patent Number 4.397.311.

Applicants have recognized the need for a cutting device having a tissue surface to abut tissue and a cutting implement movable generally parallel to the plane of the tissue surface to create a cut line that does not extend to the proximal end of the tissue abutting the device. Applicants have recognized the need for a device having a cutting implement that moves parallel to the tissue to be severed, and can be cammed towards and away from the tissue to be severed to locate the cut line relative to the device. Applicants have further recognized a need for a method of using the device to perform an anastomosis.

### Summary of the Invention

There is provided a device according to claim 1, having a tissue surface and a cutting implement movable generally parallel to the plane of the tissue surface and capable of producing a cut line that does not extend to the proximal end of tissue abutting the device, the cutting implement comprising a flexible part. The cutting implement can be cammed towards and away from tissue to be severed to control the longitudinal position of the passageway created. The device can further include surgical fasteners, or staples, for holding together the severed edges of the tissue.

### Brief Description of the Drawings

The novel features of the invention are set forth with particularity in the appended claims. The invention itself, however, as to organization together with further objects and advantages thereof, may best be understood by reference to the following description, taken in conjunction with the accompanying drawings in which:
Figure 1 is an isometric view of a surgical cutter having an a cartridge assembly according to an embodiment of the invention.
Figure 2 is an isometric view of a cartridge assembly according to an embodiment of the invention.
Figure 3 is an isometric view with a section taken along line 2-2 of Figure 1.
Figure 4 is an isometric view of a cutting implement used within the cartridge assembly of Figure 1.
Figure 5 is a section view in side elevation taken along line 2-2 of Figure 1 with the cutting implement at a proximal position within the cartridge assembly.
Figure 6 is a section view in side elevation taken along line 2-2 of Figure 1 with the cutting implement moved distally to the ascending ramp portion of the cartridge assembly.
Figure 7 is a section view in side elevation taken along line 2-2 Figure 1 with the cutting implement moved distally to the central cam portion of the cartridge assembly.
Figure 8 is a section view in side elevation taken along line 2-2 of Figure 1 with the cutting implement moved distally to the descending ramp portion of the cartridge assembly.
Figure 9 is an isometric view showing the cartridge assembly of Figure 1 used in an endoscopic linear cutter inserted into two lumens to begin a side to side anastomosis.
Figure 10 is an isometric view showing a side to side anastomosis with one lumen partially broken away to show a passageway created by a linear cutter using the cartridge assembly of Figure 1.

### Detailed Description of the Invention

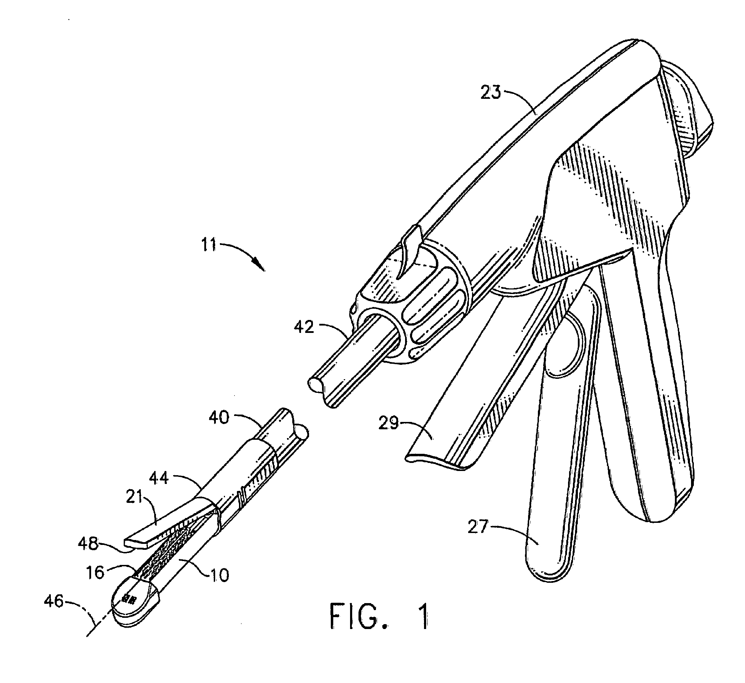

Figure 1 shows an isometric view of a surgical cutter or linear cutter 11 equipped with a cartridge assembly 10 and an anvil 21. Linear cutter 11 comprises a frame 23 capable of carrying cartridge assembly 10 and using cartridge assembly 10 to create an opening within tissue. Frame 23 of linear cutter 11 also provides a closing trigger 27 and a firing trigger 29. Closing trigger 27 and firing trigger 29 both rotate proximally to actuate mechanisms to effect tissue approximate cartridge assembly 10 and anvil 21.

Figure 1 further shows how linear cutter 11 can be constructed to manipulate tissue. Linear cutter 11 possesses a shaft 40 having a shaft proximal end 42 and a shaft distal end 44. Cartridge assembly 10 and anvil 21 can be described as two linear forks attaching to linear cutter 11 at shaft distal end 44. Cartridge assembly 10 and anvil 21 together comprise an end effector to perform work on tissue. Cartridge assembly 10 provides a housing tissue surface 16, or first tissue engaging surface, to contact tissue in use, while anvil 21 provides an anvil tissue surface 48, or second tissue engaging surface. Figure 1 depicts cartridge assembly 10 and anvil 21 open to receive tissue, however, anvil 21 may rotate towards cartridge assembly 10 until anvil tissue surface 48 and housing tissue surface 16 are substantially parallel. The two substantially parallel tissue surfaces can establish a longitudinal axis 46 parallel to and between the surfaces.

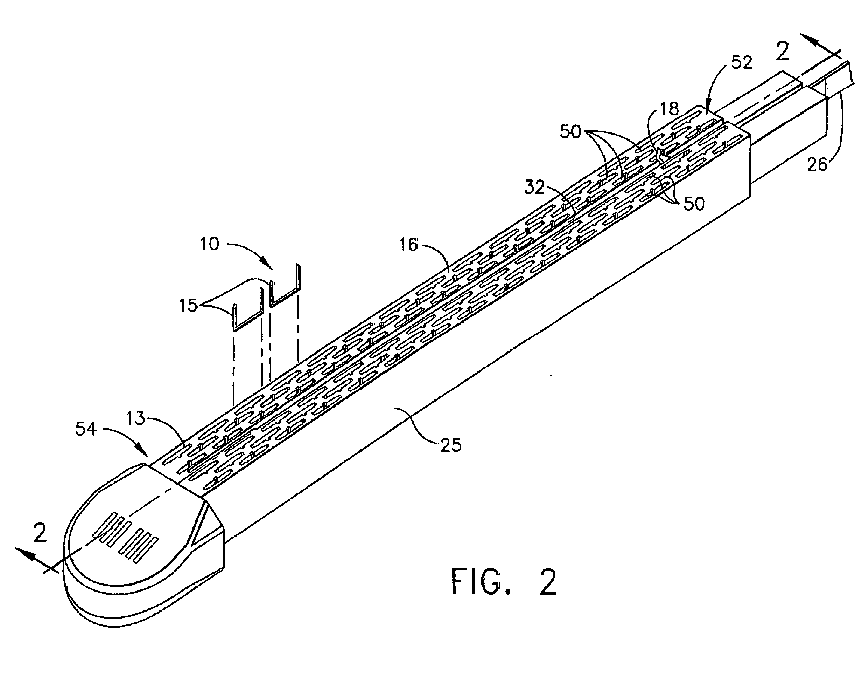

Figure 2 shows an isometric view of cartridge assembly 10. Cartridge assembly 10 has a cartridge, or a housing 25, on which housing tissue surface 16 exists to abut tissue to be cut and stapled using cartridge assembly 10. Cartridge assembly 10 further contains a plurality of tissue fasteners, or staples 15 residing in staple pockets 13. Staples 15 are arranged in at least two spaced staple rows 50, and Figure 2 depicts six staple rows 50. The staple rows 50 commence at a staple row proximal end 52 and end at a staple row distal end 54.

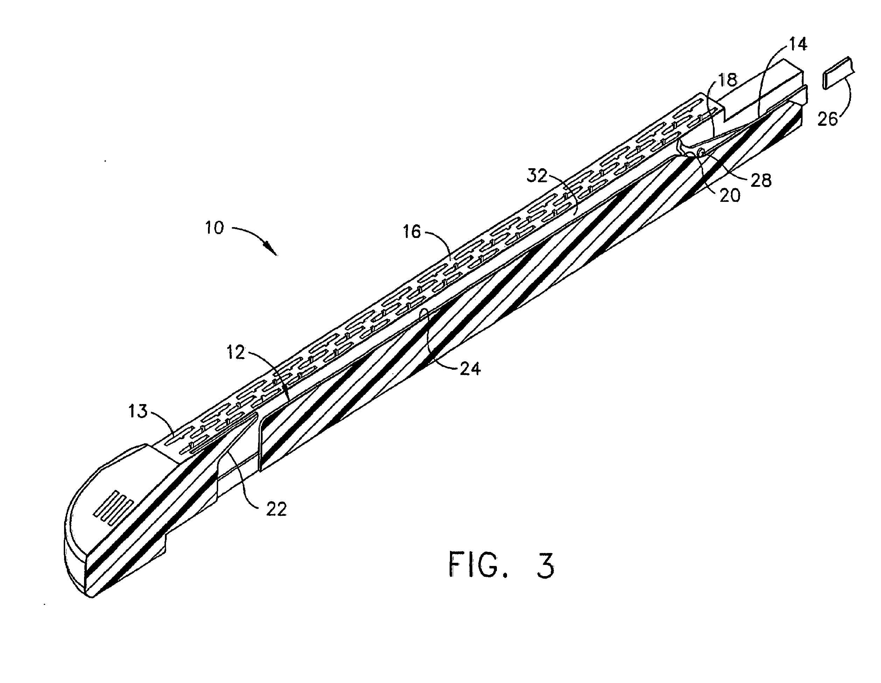

Figure 3 shows an isometric section view taken to reveal the cam surfaces 12 of cartridge assembly 10. The section view further reveals within cartridge assembly 10 a slot 32 and a cutting implement, or knife 18 which can be either part of the cartridge, or within the instrument itself. Cam surfaces 12 extend from the proximal portion of cartridge assembly 10 to a central portion of cartridge assembly 10. Cam surfaces 12 comprise a cam surface proximal portion 14, an ascending ramp portion 20, a central cam portion 24, and a descending ramp portion 22. Cam surface proximal portion 14 is separated from tissue surface 16 by a distance large enough so that when knife 18 is in the proximal portion of cartridge assembly 10 it is concealed and not exposed to any tissue adjacent tissue surface 16. Distal to cam surface proximal portion 14, an ascending ramp portion 20 extends distally and slopes towards tissue surface 16. Near the central part of cartridge assembly 10, ascending ramp portion 20 reaches its closest point to tissue surface 16. Central cam portion 24 continues distally from ascending ramp portion 20 to descending ramp portion 22. Near the distal end of cartridge assembly 10, descending ramp portion 22 slopes distally away from tissue surface 16. Descending ramp portion 22 begins nearer to tissue surface 16 than central cam portion 24, and is separated from the rest of cam surfaces 12. Cam surfaces 12 create a track to direct motion of knife 18 towards and away from tissue surface 16 and any tissue adjacent tissue surface 16. Cam surfaces 12 can be molded into cartridge assembly 10.

Figure 3 further shows a cam follower 28 extending from one side of knife 18. Cam follower 28 contacts and follows cam surfaces 12 as knife 18 advances distally. Knife actuator 26 pushes knife 18 distally. Knife actuator 26 could be, for example, a portion of any endoscopic linear cutter having a frame 23 (Figure 1) into which cartridge assembly 10 could be inserted, such as a linear cutter 11 depicted in Figure 1. Knife actuators and other means for actuating the firing and closing of the instrument are well known to those skilled in the art. Examples of such are disclosed in United States Patent Number 5,597,107, issued on January 28, 1997.

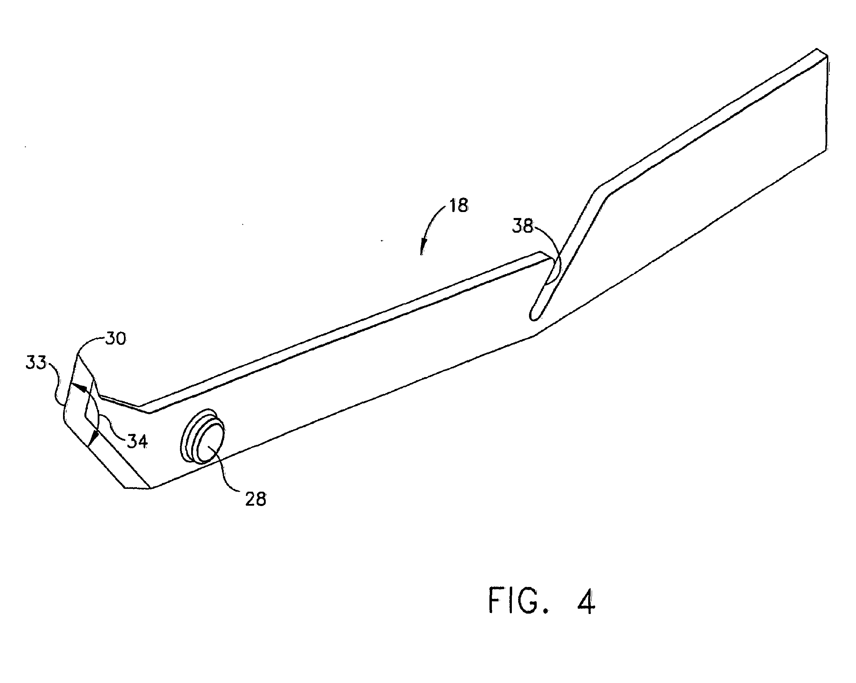

Figure 4 is an isometric view of knife 18. In addition to cam follower 28, knife 18 incorporates knife slot 38 to allow knife 18 to flex. The distal end of knife 18 carries a knife edge 33 and a knife tip 30. Knife edge 33 is capable of slicing tissue, while knife tip 30 can pierce tissue. Knife edge 33 is placed around an angle 34 at the distal end of knife 18.

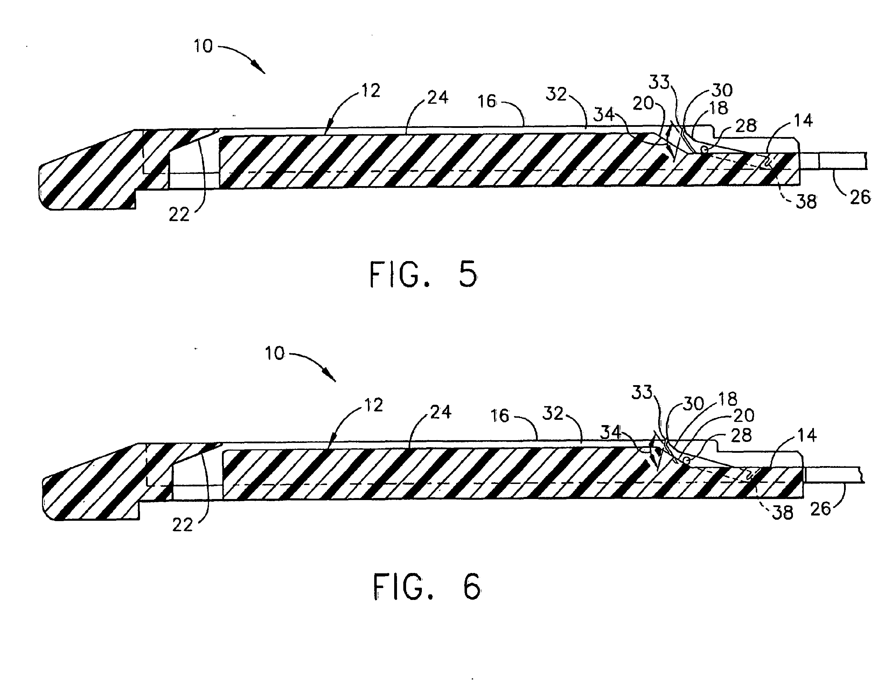

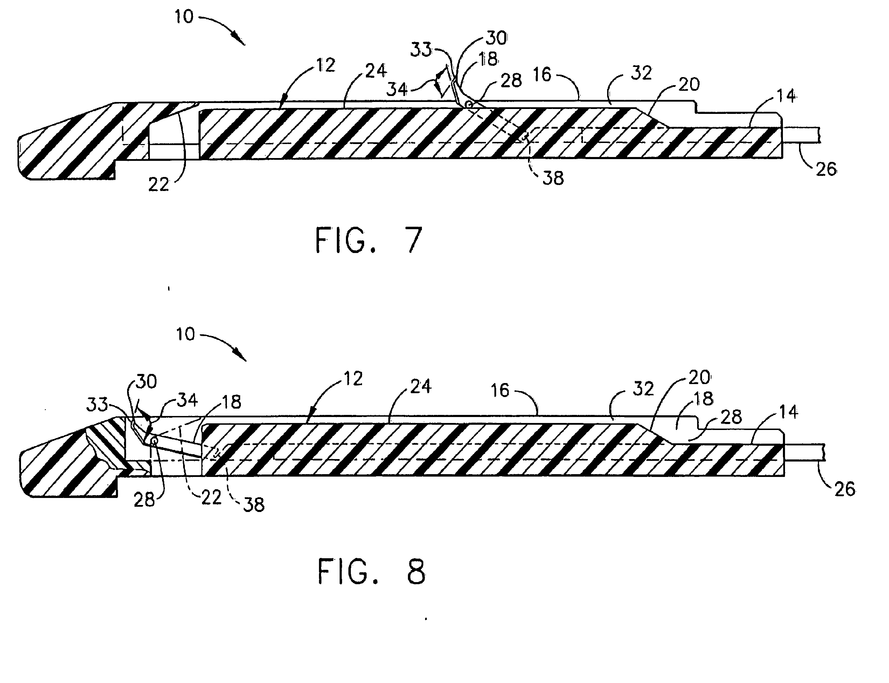

Figure 5 shows cartridge assembly 10 with knife 18 placed forward to a point proximal of the intersection of ascending ramp portion 20 and central cam portion 24. Cartridge assembly 10 can be inserted into linear cutter 11. Inserting cartridge assembly 10 and pulling firing trigger 29 (Figure 1) moves knife actuator 26 forward causing knife 18 to move through slot 32. Cam follower 28 contacts cam surfaces 12 to urge knife 18 towards and away from tissue surface 16 as knife 18 moves distally generally parallel to tissue surface 16. Cam follower 28 first contacts cam surface proximal portion 14. While cam follower 28 contacts cam surface proximal portion 14, knife 18 avoids tissue surface 16 and does not cut tissue. As knife 18 moves distally, cam follower 28 contacts ascending ramp portion 20, which drives knife 18 towards tissue surface 16. The distal portion of knife 18 flexes towards tissue surface 16 at the flexible portion or slot 38 while the proximal portion of knife 18 remains in axial alignment with knife actuator 26. When knife tip 30 is exposed from tissue surface 16, knife tip 30 pierces tissue adjacent tissue surface 16 and begins making a cut. Because knife edge 33 is placed around angle 34, the portion of the angled edge nearest the tissue faces and cuts tissue as knife 18 moves towards tissue. Cam follower 28 reaches central cam portion 24 and knife 18 ceases to move towards tissue surface 16. Knife slot 38 is designed to allow knife 18 to flex to a position shown in Figure 7. Knife 18 continues to cut tissue abutting tissue surface 16 as knife 18 travels proximally. Knife 18 cuts the tissue in a direction parallel to tissue surface 16. Knife 18 travels forward parallel to tissue surface 16 until knife 18 reaches descending ramp portion 22. Descending ramp portion 22 contacts cam follower 28 and drives knife 18 away from tissue surface 16, placing knife 18 into a position depicted by Figure 6. Knife 18 moves away from tissue and ceases to cut. Knife 18 is concealed from tissue abutting tissue surface 16. The mechanism of linear cutter 11 will then cause knife actuator 26 to retract. The configuration of cartridge assembly 10 can cause a cut line that does not extend to the proximal end of the cartridge. The configuration of cartridge assembly 10 causes knife 18 to emerge from housing tissue surface 16 only between staple row proximal end 52 and staple row distal end 54 (Figure 2). Using cartridge assembly 10 in linear cutter 11 causes linear cutter 11 to produce in tissue abutting tissue surface 16 a cut line that does not extend proximally to the proximal portion of the tissue. Knife 18 cuts only between staple row proximal end 52 and staple row distal end 54.

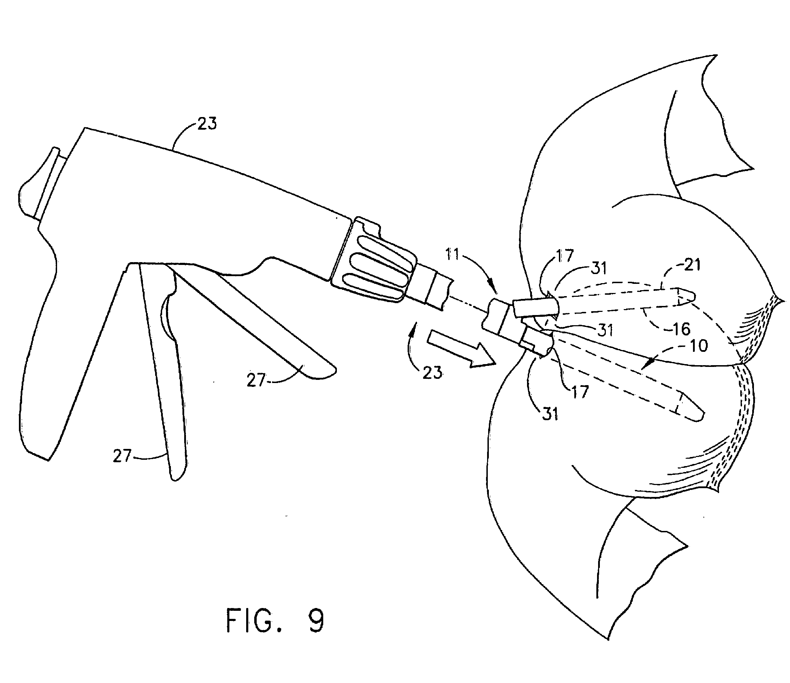

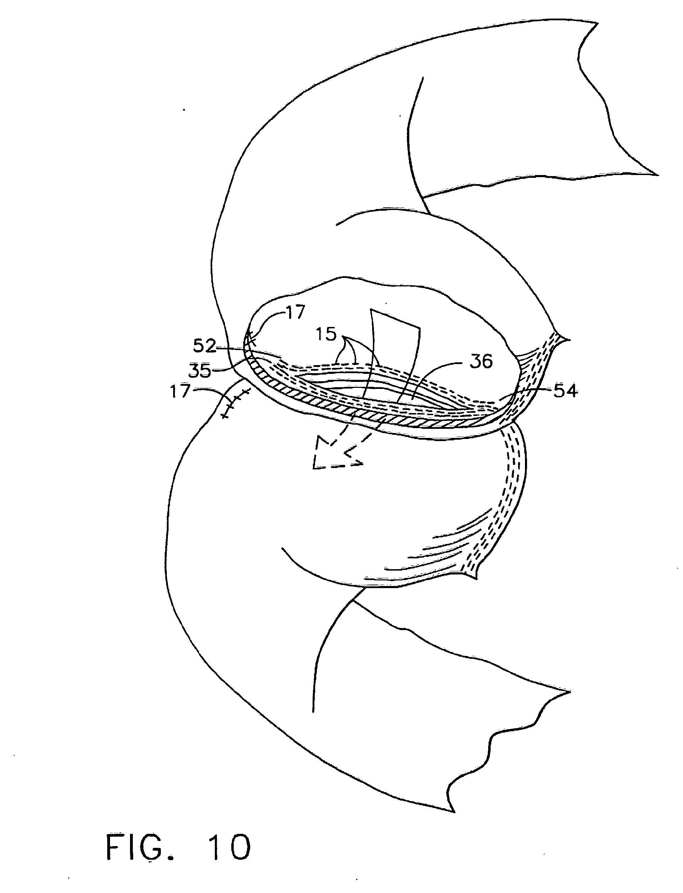

Figures 9 and 10 show a side-to-side anastomosis of two body lumens. In a side-to-side anastomosis of two body lumens, a surgeon can use linear cutter 11 equipped with cartridge assembly 10 and anvil 21. Figure 9 shows that the surgeon creates two small openings 17, one each in each body lumen, and inserts anvil 21 of linear cutter 11 in a first lumen and the cartridge assembly 10 of linear cutter 11 into the second lumen. Closing anvil 21 to cartridge assembly 10 compresses a portion of a wall of each body lumen into a plane adjacent tissue surface 16. The portions of the edges of the openings clamped between anvil 21 and cartridge assembly 10 become the proximal tissue end 31. The surgeon then can fire linear cutter 11 creating a cut line and a passageway 36 in the tissue clamped between anvil 21 and cartridge assembly 10 of linear cutter 11 by moving knife 18 generally parallel to tissue surface 16 and the plane of the compressed tissue. Knife 18 begins the cut line at a point distal to proximal tissue end 31, and cuts tissue only between staple row proximal end 52 and staple row distal end 54. The surgeon need close only two small openings 17 to complete the procedure, creating passageway 36.

Figure 10 shows the resultant passageway 36 created. Linear cutter 11 has inserted staples 15 into tissue adjacent the cut line to adhere the wall of one lumen to the wall of the other lumen, to control bleeding, and to prevent leakage of lumen contents to the lumen's exterior. A distance of uncut tissue 35 will exist in the lumen walls between the openings and the beginning of the cut. Sutures or staples may be used to close the two openings 17. Matter, such as digestive fluids or ingesta, may pass through passageway 36. If the anastomosis were a vascular anastomosis, blood may pass through passageway 36.

It will be recognized that equivalent structures may be substituted for the structures illustrated and described herein and that the described embodiment of the invention is not the only structure which may be employed. As one example of an equivalent structure that may be used, linear cutter 11 may be any linear cutter, useful for open or endoscopic surgery, having a frame 23 to which the cartridge assembly 10 may be attached. Linear cutter 11 may further include alternate equipment to adhere tissue, such as radiofrequency energy appliers, or laser energy appliers. As a further example of an equivalent structure, knife 18 may be any cutting implement such as a disk with an edge about its circumference. The disk may be advanced distally, substantially parallel to tissue surface 16, as it is cammed towards and away from tissue surface 16.As a further example of an equivalent method that may be used, the method of joining two lumens can be useful in joining many types of internal body lumens such as the stomach, small bowel, urinary vessels, or blood vessels. Such a method becomes useful in joining small intestine to small intestine, or small intestine to stomach, as is often done in gastric bypass surgery.

Another method of cutting substantially parallel to a tissue surface can be to rotate a knife so that the cutting edge faces tissue at an area where the proximal end of the cut should begin. The knife, for example, can have a profile such that it lies within a slot in the cartridge below the tissue surface. The profile of the knife is such that the cutting edge is longer, and hence taller to extend above the tissue surface of the cartridge when rotated to cause the cutting edge to face tissue. A pivot point, such as a pin, can be placed within the knife slot where the proximal end of the cut in tissue is desired. A push arm pushes the knife distally, and the pin intercepts the distally moving knife. The knife can then rotate from a profile in which it lies below the surface of the tissue to a second position presenting the cutting edge to tissue. The push arm engages the knife at a point a distance away from the pivot point, or pin. The force exerted by the push arm and the reactive force by the push pin create, because of the separation in distance from the two points, a moment tending to rotate the knife. After rotation, the knife can travel distally while the cutting edge faces tissue, to cut tissue and create an otomy.

While preferred embodiments of the present invention have been shown and described herein, it will be obvious to those skilled in the art that such embodiments are provided by way of example only. Numerous variations, changes, and substitutions will now occur to those skilled in the art. For example, as would be apparent to those skilled in the art, the disclosures herein have equal application in robotic-assisted surgery. In addition, it should be understood that every structure described above has a function and such structure can be referred to as a means for performing that function. Accordingly, the invention is understood not to be limited to the details of structure and operation shown and described in the specification and drawings.

## Claims

1. A device (11) for creating an anastomosis between two layers of tissue, said device comprising:
a) a shaft (40) having proximal (42) and distal (44) ends, and an end effector attached to said distal end of said shaft, said end effector comprising a first (10) and a second (21) elongated fork having distal and proximal ends and a longitudinal axis (46) therebetween, said first fork (10) having a first tissue engaging surface (16) and said second fork (21) having a second tissue engaging surface (48), said end effector having an open position wherein at least said distal end of the forks are spaced from one another, and a closed position, wherein said tissue engaging surfaces are closely adjacent, said end effector further including a means for applying at least two spaced part rows (50) of tissue fasteners (15) onto tissue parallel to said longitudinal axis;
b) a cutting implement (18), said cutting implement movable between said two spaced apart rows of fasteners in a direction parallel to said longitudinal axis (46) of said forks; and
c) a means for actuating said cutting implement such that said cutting implement (18) cuts only between a proximal end (52) and a distal end (54) of said rows of tissue fasteners,
d) a track (12)
**characterised in that**
said cutting implement (18) comprises a flexible part (38), said flexible part being adapted to bend to allow a first portion of said cutting implement to follow said track to cut only between the proximal end (52) and the distal end (54) of said rows of tissue fasteners.

2. A device according to claim 1 further comprising said track (12) within said first fork (10), said track capable of directing said cutting implement (18) along a cut line beginning from a point distal to a proximal tissue end (31).

3. A device according to claim 2 wherein said track (12) further comprises an ascending portion (20) for camming said cutting implement (18) towards said first tissue engaging surface (16) to expose said cutting implement.

4. A device according to claim 3 wherein said track (12) further comprises a descending portion (22) for camming said cutting implement (18) away from said first tissue engaging surface (16) to conceal said cutting implement.

5. A device according to claim 1 wherein said cutting implement (18) further comprises a piercing tip (30) that emerges perpendicular to said tissue engaging surface (16) when said cutting implement is exposed.

6. A device according to claim 5 wherein said cutting implement (18) further comprises a cutting surface (33) that subtends an angle (34) around a distal end of said cutting implement.

7. A device according to any one of claims 2 to 6 further comprising a cam follower (28) extending from one side of said cutting implement (18), said cam follower (28) contacting with said track (12) to urge said cutting implement (18) towards and away from said first tissue surface (16) as said cutting implement (18) moves in a direction parallel to said first tissue surface (16).

8. A device according to any one of claims 1 to 7 wherein the flexible part (38) comprises a slot to allow said first portion (28) of said cutting implement to bend to follow said track (12).

## Patentansprüche

1. Instrument (11) zum Erzeugen einer Anastomose zwischen zwei Gewebsschichten, wobei das Instrument Folgendes umfasst:
a) einen Schaft (40), welcher ein proximales (42) und ein distales (44) Ende und einen Endeffektor aufweist, welcher an dem distalen Ende des Schaftes befestigt ist, wobei der Endeffektor ein erstes (10) und ein zweites (21) gestrecktes Gabelelement umfasst, welche ein distales und ein proximales Ende und eine Längsachse (46) dazwischen aufweisen, wobei das erste Gabelelement (10) eine erste gewebeeingreifende Oberfläche (16) aufweist und das zweite Gabelelement (21) eine zweite gewebeeingreifende Oberfläche (48) aufweist, wobei der Endeffektor eine offene Position, in welcher zumindest die distalen Enden der Gabelelemente voneinander beabstandet sind, und eine geschlossene Position aufweist, in welcher die gewebeeingreifenden Oberflächen nahe benachbart sind, wobei der Endeffektor weiterhin ein Mittel aufweist, um zumindest zwei voneinander beabstandete Reihen (50) von Gewebebefestigungselementen (15) auf Gewebe parallel zu der Längsachse anzusetzen;
b) ein Schneidewerkzeug (18), wobei das Schneidewerkzeug zwischen den zwei voneinander beabstandeten Reihen von Befestigungselementen in eine Richtung parallel zu der Längsachse (46) der Gabelelemente beweglich ist; und
c) ein Mittel zum Betätigen des Schneidewerkzeugs, sodass das Schneidewerkzeug (18) nur zwischen einem proximalen Ende (52) und einem distalen Ende (54) der Reihen von Gewebebefestigungselementen schneidet,
d) eine Führung (12)
**dadurch gekennzeichnet, dass**
das Schneidewerkzeug (18) einen flexiblen Teil (38) umfasst, wobei der flexible Teil eingerichtet ist für ein Biegen, um es einem ersten Teilbereich des Schneidewerkzeugs zu erlauben, der Führung zu folgen, um nur zwischen dem proximalen Ende (52) und dem distalen Ende (54) der Reihen von Gewebebefestigungselementen zu schneiden.

2. Instrument gemäß Anspruch 1, welches weiterhin umfasst, dass sich die Führung (12) innerhalb des ersten Gabelelements (10) befindet, wobei die Führung geeignet ist, das Schneidewerkzeug (18) entlang einer Schnittlinie zu führen, welche von einem Punkt beginnt, welcher distal zu einem proximalen Gewebeende (31) ist.

3. Instrument gemäß Anspruch 2, wobei die Führung (12) weiterhin einen ansteigenden Teilbereich (20) für ein Ineingriffbringen des Schneidewerkzeugs (18) auf die erste gewebeeingreifende Oberfläche (16) zu umfasst, um das Schneidewerkzeug freizulegen.

4. Instrument gemäß Anspruch 3, wobei die Führung (12) weiterhin einen absteigenden Teilbereich (22) für ein Ineingriffbringen des Schneidewerkzeugs (18) von der ersten gewebeeingreifenden Oberfläche (16) weg umfasst, um das Schneidewerkzeug zu verdecken.

5. Instrument gemäß Anspruch 1, wobei das Schneidewerkzeug (18) weiterhin eine Durchstechspitze (30) umfasst, welche senkrecht zu der gewebeeingreifenden Oberfläche (16) austritt, wenn das Schneidewerkzeug freigelegt ist.

6. Instrument gemäß Anspruch 5, wobei das Schneidewerkzeug (18) weiterhin eine Schneidefläche (33) umfasst, welche einen Winkel (34) um ein distales Ende des Schneidewerkzeugs herum einschließt.

7. Instrument gemäß einem der Ansprüche 2-6, welches weiterhin ein Eingriffsglied (28) umfasst, welches sich von einer Seite des Schneidewerkzeugs (18) erstreckt, wobei das Eingriffsglied (28) in Kontakt mit der Führung (12) ist, um das Schneidewerkzeug (18) auf die erste Gewebeoberfläche (16) zu und davon weg zu treiben, während sich das Schneidewerkzeug (18) in eine Richtung parallel zu der ersten Gewebeoberfläche (16) bewegt.

8. Instrument gemäß einem der Ansprüche 1-7, wobei der flexible Teil (38) einen Schlitz umfasst, um dem ersten Teilbereich (28) des Schneidewerkzeugs ein Biegen zu ermöglichen, um der Führung (12) zu folgen.

## Revendications

1. Dispositif (11) de création d'une anastomose entre deux couches de tissus, ledit dispositif comprenant :
a) une tige (40) ayant des extrémités proximale (42) et distale (44) et un effecteur terminal fixé à ladite extrémité distale de ladite tige, ledit effecteur terminal comprenant une première (10) et une deuxième (21) fourche allongée ayant des extrémités distale et proximale et un axe longitudinal (46) entre elles, ladite première fourche (10) ayant une première surface de mise en prise du tissu (16) et ladite deuxième fourche (21) ayant une deuxième surface de mise en prise du tissu (48) ledit effecteur terminal ayant une position ouverte dans laquelle au moins lesdites extrémités distales des fourches sont espacées l'une de l'autre, et une partie fermée dans laquelle lesdites surfaces de mise en prise du tissu sont étroitement adjacentes, ledit effecteur terminal comprenant en outre un moyen d'application d'au moins deux rangées partielles espacées (50) d'agrafes tissulaires (15) sur le tissu parallèle audit axe longitudinal ;
b) un instrument coupant (18), ledit instrument coupant pouvant se déplacer entre lesdites deux rangées espacées d'agrafes dans une direction parallèle audit axe longitudinal (46) desdites fourches ; et
c) un moyen d'actionnement dudit instrument coupant de telle sorte que ledit instrument coupant (18) coupe uniquement entre une extrémité proximale (52) et une extrémité distale (54) desdites rangées d'agrafes tissulaires,
d) une piste (12)
**caractérisé en ce que** ledit instrument coupant (18) comprend une partie flexible (38), ladite partie flexible étant adaptée pour se courber pour permettre à une première partie dudit instrument coupant de suivre ladite piste pour couper uniquement entre l'extrémité proximale (52) et l'extrémité distale (54) desdites rangées d'agrafes tissulaires.

2. Dispositif selon la revendication 1, comprenant en outre ladite piste (12) dans ladite première fourche (10), ladite piste étant capable de diriger ledit instrument coupant (18) le long d'une ligne de coupe commençant d'un point distal par rapport à une extrémité de tissu proximal (31).

3. Dispositif selon la revendication 2, dans lequel ladite piste (12) comprend en outre une partie ascendante (20) pour diriger ledit instrument coupant (18) vers ladite première surface de mise en prise du tissu (16) pour exposer ledit instrument coupant.

4. Dispositif selon la revendication 3, dans lequel ladite piste (12) comprend en outre une partie descendante (22) pour diriger ledit instrument coupant (18) à distance de ladite première surface de mise en prise du tissu (16) pour cacher ledit instrument coupant.

5. Dispositif selon la revendication 1, dans lequel ledit instrument coupant (18) comprend en outre une pointe de perforation (30) qui dépasse perpendiculairement à ladite surface de mise en prise du tissu (16) lorsque ledit instrument coupant est exposé.

6. Dispositif selon la revendication 5, dans lequel ledit instrument coupant (18) comprend en outre une surface coupante (33) qui sous-tend un angle (34) autour d'une extrémité distale dudit instrument coupant.

7. Dispositif selon l'une quelconque des revendications 2 à 6, comprenant en outre un galet de came (28) s'étendant d'un côté dudit instrument coupant (18) vers et à distance de ladite première surface (16) lorsque ledit instrument coupant (18) se déplace dans une direction parallèle à ladite première surface de tissu (16).

8. Dispositif selon l'une quelconque des revendications 1 à 7, dans lequel la partie flexible (38) comprend une fente pour permettre à ladite première partie (28) dudit instrument coupant de se courber pour suivre ladite piste (12).
